# EUROPEAN PATENT SPECIFICATION

(11) **EP 2 259 825 B1**
(45) Date of publication and mention of the grant of the patent: **26.05.2021**
(21) Application number: 09731360.5
(22) Date of filing: 07.04.2009
(51) Int. Cl.: A61M 16/00, A61M 16/04

(54) **TRACHEAL TUBES AND A METHOD OF MAKING THE SAME**
TRACHEALRÖHREN UND VERFAHREN ZU IHRER HERSTELLUNG
TUBES TRACHÉAUX ET LEUR PROCÉDÉ DE FABRICATION

(30) Priority: 08.04.2008 US 78920
(43) Date of publication of application: 15.12.2010
(73) Proprietor: Smiths Group plc, London SW1E 5JL (GB)
(72) Inventor: COATES, Daniel, Jay, Ogen Dunes IN 46368 (US)
(74) Representative: Hedges, Martin Nicholas
(86) International application number: PCT/US2009/002159
(87) International publication number: WO 2009/126248

(56) References cited:
- US-A- 3 889 688
- US-A- 4 328 056
- US-A- 4 419 095
- US-A- 4 419 095
- US-A- 5 845 992
- US-A- 6 143 228
- US-A1- 2007 295 337
- US-A1- 2007 295 337
- US-A1- 2008 000 482
- US-A1- 2008 000 482
- US-A1- 2008 021 386
- US-A1- 2008 072 911

## Description

The present invention relates to tracheal tubes and in particular to a tracheostomy tube with an inverted cuff.

### Background of the Invention

In the making of cuffed tracheal tubes, for example tracheostomy tubes, and in particular those tracheostomy tubes that are made from silicone, the silicone cuffs that are attached to those tubes are usually formed by injection molding where a mold consists of an upper cover and a lower base. As a result, there are often flash lines or parting lines formed along the lengths of the cuffs where the upper portion of the mold meets the lower portion of the mold. The flash line results from excess silicone that seeps out where the upper and lower portions of the mold meet. Although efforts have been made as part of the manufacturing process to eliminate the flash lines at the cuffs, the unsightly flash lines nonetheless are readily seen and may be felt by the patient or be snagged when the tracheal tube is inserted into the patient. Another problem that may occur is that the flash or excess material that form the flash lines may fall off while the tracheal tube is in the patient.

US 3889688, which is considered to represent the closest prior art, discloses a tracheal tube comprising a tubular shaft, an inflatable sealing cuff manufactured with an original inner surface and an original outer surface, the cuff including a collar at each end attached proximate to a patient end of said shaft at space locations and lumen integrally formed along at least a portion of said shaft with a patient end located inside said cuff for enabling fluid to be input from a machine and of the lumen into said cuff when inflating said cuff.

According to a first aspect of the present invention there is provided a method of manufacturing a tracheal tube according to claim 1.

The present invention further provides a tracheal tube according to claim 6.

To minimize discomfort to the patient and also provide an appearance of smoothness, the present invention cuffed tracheal tube is made with the cuff inverted or turned inside-out so that the smooth interior surface of the cuff is exposed to the environment while the original exterior surface of the cuff with the flash lines is attached, at its end collars, to the distal end of the tubular shaft of the tracheal tube. By inverting the cuff, the flash line(s) that formed at the outside surface of the cuff is hidden from view and also will not cause discomfort to the patient as the tracheal tube is inserted to the patient. Moreover, any flash or excess material that may detach from the cuff at the flash line is trapped within the cuff, and therefore not fall into the patient. Furthermore, by having a completely smooth outer cuff surface, upon inflation, a better seal is formed between the cuff and the trachea of the patient. Also, the smooth cuff surface prevents potential secretion buildup on the cuff. So, too, a smooth cuff surface eases or facilitates the cleaning of the tracheal tube.

The present invention will become more apparent and the invention itself will be best understood by reference to the following description of the invention taken in conjunction with the following drawings, wherein:
Fig. 1 is a side view of a cuff for a tracheal tube after it has been removed from its mold;
Fig. 2 is a cross-section view of the Fig. 1 cuff showing the interior surface of the cuff;
Fig. 3 is a perspective view of the cuff shown in Figs. 1 and 2 having been inverted so that the original inside surface is now exposed to the environment;
Fig. 4 is a side view of a tracheal tube, for example a tracheostomy tube of the present invention;
Fig. 5 is a top view of the tracheostomy tube of Fig. 4;
Fig. 6 is a top view of the lower half of a mold with a core used for making a plurality of cuffs of the instant invention positioned therein;
Fig. 7 is a side view of the lower portion of the mold as shown in Fig. 6 relative to its upper portion; and
Fig. 8 is a perspective view of the lower portion of the mold of Fig. 6.

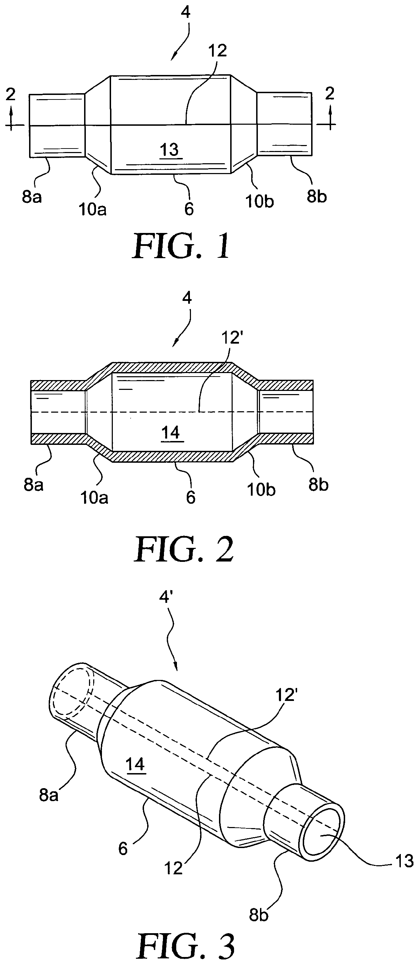

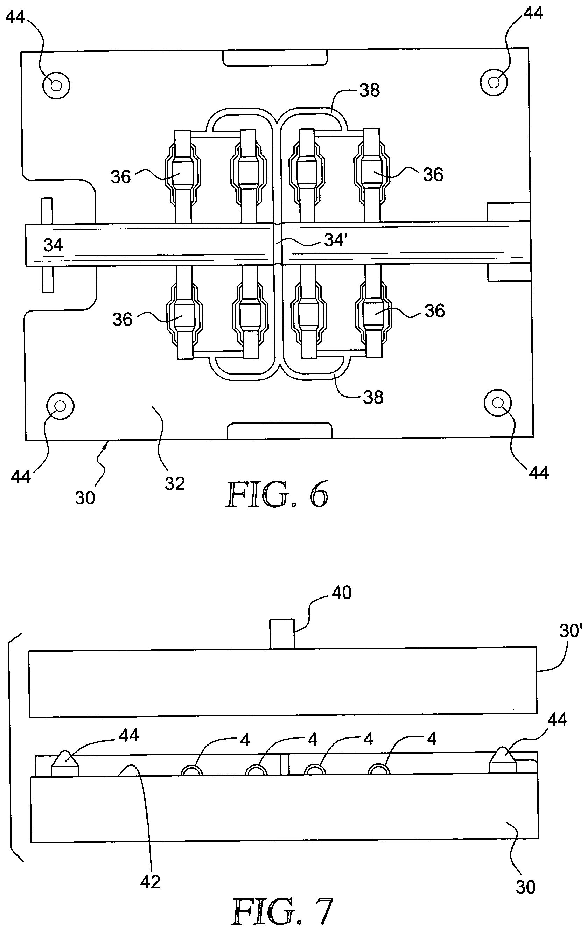

With reference to Fig. 1, a cuff 4 made from injection molding is shown. The cuff may be made of silicone rubber or other material such as PVC and has an inflatable main portion 6 and two collars 8a and 8b at its ends. Collars 8a and 8b extend from main portion 6 via respective transition portions 10a and 10b. When removed from the mold, flash lines are usually formed longitudinally along opposite sides at the outer surface of cuff 4 due to the molding flash or excess material attached to the cuff at the junction or plane where the upper and lower portions of the mold meet, as will be further explained with respect to Figs. 6-8. For the embodiment where the cuff is molded from silicone, the excess material is silicone.

Attempts have been made to remove this excess material, or molding flash. But in the case of a silicone cuff, due to its thin layer and soft texture, short of tearing or causing a hole, there remains at the cuff at least a portion of the flash line 12 that could not be removed. The flash line opposite flash line 12 in the back of cuff 4 shown in Fig. 1 is shown as a dotted line 12' in the cross-sectional view of cuff 4 in Fig. 2. The exterior or outer surface of cuff 4 is designated 13 in Fig. 1.

With reference to Fig. 2, the cross-sectional view 2--2 of cuff 4 is shown. As was noted earlier, the flash line 12' on the exterior surface 13 of cuff 4 away from the reader is designated 12'. Further as shown, the interior or inner surface 14 of cuff 4 is smooth and unmarked by any defect from the molding process.

Fig. 3 is a perspective view of the cuff shown in Fig. 1 having been inverted or turned inside-out, so that its original inner surface 14 is now exposed to the environment while its original outer surface 13 is now the inner surface of the cuff. For ease of discussion, the cuff shown in Fig. 3 is designed 4'. All other designations remain the same as those in Fig. 1. As shown, flash lines 12 and 12' are now within the interior surface of cuff 4', so that the exterior surface (original inner surface 14), of cuff 4' is now completely smooth.

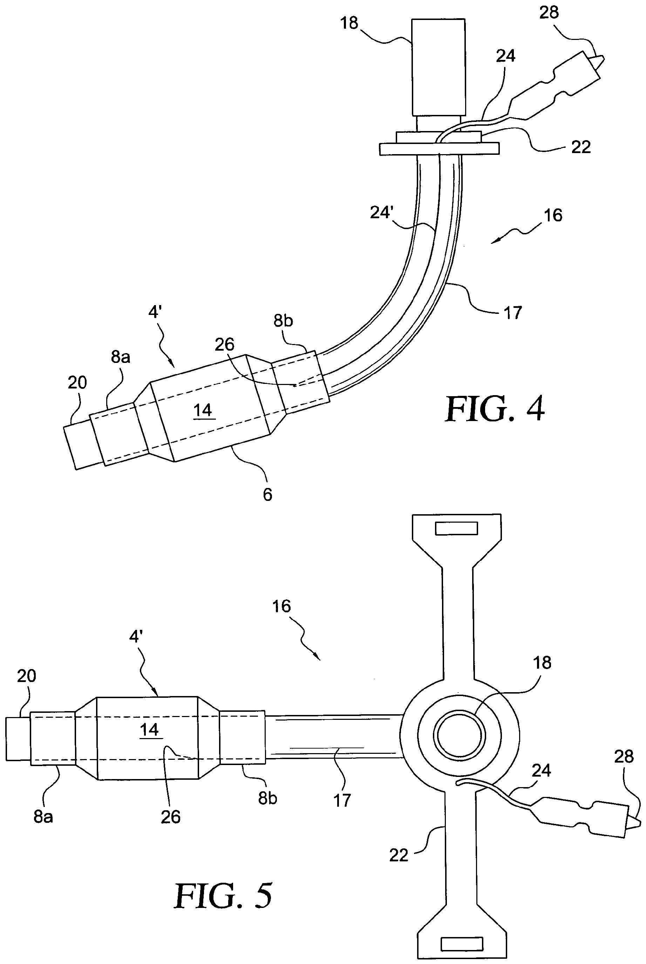

Fig. 4 shows an exemplar tracheal tube, for example a tracheostomy tube 16. Tracheostomy tube 16 is formed by an elongate tubular shaft 17 that has a patient end 20 and a machine end 18. Shaft 17 may be a straight tube, or may be curved along a part of its length. Proximate to the patient end 20, cuff 4' is attached by means of its glue bands or collars 8a and 8b at spaced locations proximate to the patient end 20. The tracheostomy tube for this invention is preferably made also from silicone and in the preferred embodiment is injection molded as a single integral unit to have a flange 22, best shown in Fig. 5, that allows the tracheotomy tube 16 to be attached to the patient. For this embodiment of tracheostomy tube 16, the inverted cuff 4' is a TTS (Tight To Shaft) fit to shaft 17, where the cuff lies close to the shaft when it is in its non-inflated resting state. This minimizes any snag to the cuff when the tracheostomy tube is placed into a patient.

Attached to tracheostomy tube 16 at its machine end 18 close to flange 22 is a lumen or tube 24 that is integrated along a path 24' substantially along the length of shaft 17 of the tracheostomy tube, with its patient end 26 residing within main portion 6 of cuff 4'. By connecting a syringe or other input means to its machine end 28, a fluid such as for example air or a liquid, possibly sterile water, may be fed through lumen 24 into cuff 4' to inflate cuff 4'.

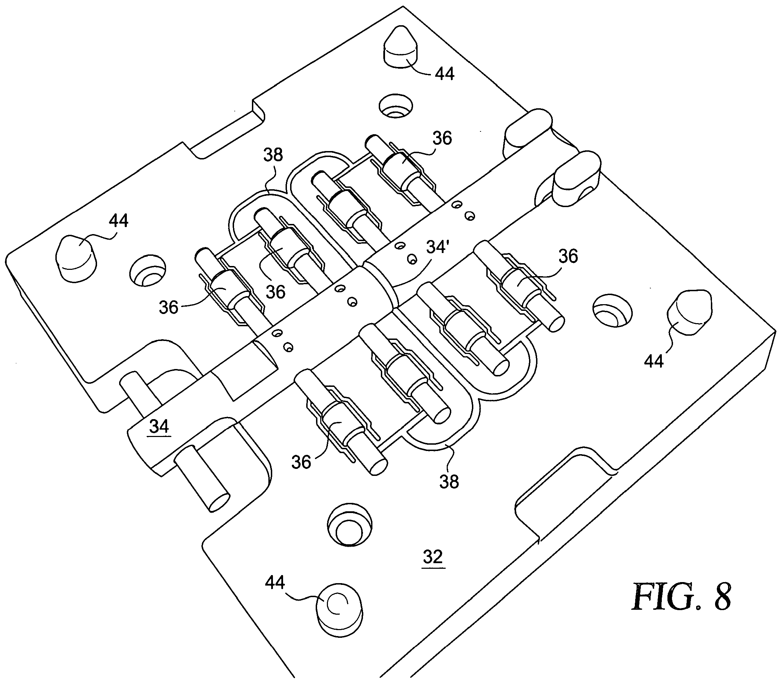

With reference Figs. 6 and 8, the lower portion of the mold for making the cuff of the instant invention is shown. As illustrated, the lower mold 30 is made of either hardened steel or aluminum, or some metal alloy such as beryllium and copper. Lower mold 30 is shown to include a bed 32 onto which a central mold core 34 that has attached thereto a plurality of cuff-shaped branch cores 36 is positioned. Central core 34 is removable from bed 32 so that the silicone rubber cuffs formed on the cuff-shaped branch cores 36 may be extracted. As further shown, there is a groove 34' formed at the center of core 34, and there are a number of grooves or runners 38 formed in bed 32 of lower mold 30. Runners 38 allow the silicone material, poured into the mold as a liquid resin from a sprue or input port 40 shown in the upper mold 30' of Fig. 7, to flow around the cuff-shaped branch cores 36 to form the plurality of cuffs. This of course is done only after the upper mold 30' has been placed over and secured to the lower mold 30. Upper mold 30' is guided onto lower mold 30 by guide pins 44.

When the silicone liquid resin is injected into the mold, by way of sprue 40 at upper mold 30', the liquid silicone resin first flows along groove 34' at core 34 and then flows along the runners 38 to surround the various cuff-shaped branch cores 36. After curing, upper mold 30' is lifted off the lower mold 30 and central core 34 is removed from lower mold 30. The individual cuffs formed at the branch cores 36 could then be removed. Note that since a mold is made up of upper and lower portions, upon the positioning of upper mold 30' over the lower mold 30, during the injection and curing processes, there usually is formed a parting line at each side of each of the cuffs at the plane where the bottom surface of upper mold 30' meets the upper surface of the lower mold 30. These parting lines result in the flash lines 12 and 12' shown in Figs. 1-3 and discussed above. The parting lines or flash lines for the different cuffs, designated 4, formed at the branch cores 36 are best shown in Fig. 7 along the plane 42.

By inverting the cuffs retrieved from the cuff-shaped branch cores 36, the smooth inner surface 14 is now exposed to the environment. By attaching cuff 4' to shaft 17 at spaced locations with an adhesive, for example a liquid silicone RTV (Room Temperature Vulcanization), the flash lines that, prior to the instant invention would cause discomfort to the patient and render the tracheostomy tube unsightly, is hidden from view. Moreover, the flash, or excess silicone material, that could fall off from the flash line is now trapped within the interior of cuff 4', more specifically the space defined by the interior surface of cuff 4' and the outer surface of shaft 17covered by cuff 4'. Further, the smooth cuff surface prevents potential secretion buildup on the cuff and facilitates the cleaning of the tracheotomy tube after its removal from the patient. Furthermore, the flash lines in the interior surface of the cuff 4', which due to the TTS configuration, may establish an air passage between the inner surface 13 of the cuff 4' and the outer surface of the shaft 17, to thereby assist in the inflation of cuff 4'. In other words, as the cuff is almost a parallel sleeve to the shaft for a TTS type tracheostomy or tracheal tube, in the case where the cuff is not inverted, the smooth inner surface of the cuff may come into contact with and be stuck to the outer circumferential surface of the shaft so that inflation of the cuff may be more difficult.

With the inverting of the cuff, external features other than the flash lines may be deliberately molded onto the cuff so that, when the cuff is inverted, those features on the cuff surface that now faces the outer circumferential surface of the shaft will prevent the cuff from sticking to the outer circumferential surface of the shaft. Further, those external features may include circumferential ribs formed proximate to or at the collars of the cuff so that when the "as molded" cuff is turned inside out, those ribs (separately or in combination with corresponding ribs or other features formed on the opposing wall surface of the shaft) would form glue dams that restrict the flow of adhesive or solvent from seeping from where the collars of the cuff are glued to the wall of the shaft out to the portion of the cuff that is to be inflated.

While the present invention is disclosed with reference to tracheal or tracheostomy tubes and cuffs that are made from silicone, it should be appreciated that the instant invention is also applicable to cuffed tracheal or tracheostomy tubes and cuffs or other medical tubes and cuffs made from materials that may also cause parting or flash lines for the cuffs. Moreover, instead of injection molding, the present invention cuff may be manufactured by other molding methods including for example blow molding. Furthermore, the inverting of the cuff allows for a smooth core center or core pin, which in turn leads to easy removal of the core pin from the molded cuff.

## Claims

1. A method of manufacturing a tracheal tube, comprising the steps of:
molding a tubular shaft;
molding a cuff including two collars, one at each end of the cuff;
inverting said cuff so that its original inner surface becomes the surface that is exposed to the environment and its original outer surface forms the interior surface of said cuff;
attaching the two collars of said cuff proximate to a patient end of said shaft at spaced locations; and
integrally forming a lumen along at least a portion of said shaft with its patient end located inside said cuff to enable said cuff to be inflated when a fluid is input to the machine end of said lumen.

2. Method of claim 1, wherein said cuff is injection molded from silicone.

3. Method of claim 1 or claim 2, wherein said shaft is injection molded from silicone.

4. Method of any of the preceding claims, wherein said cuff is attached to said shaft by bonding the collars to said shaft using a liquid silicone RTV (room temperature vulcanization) adhesive, the bonded cuff trapping material that may separate from a flash line formed at the original outer surface of said cuff.

5. Method of any of the preceding claims, further comprising the step of integrally molding a flange proximate to a machine end of said shaft so that said shaft and said flange are formed as a unitary single piece.

6. A tracheal tube (16) manufactured according to the method of any of the preceding claims, the tracheal tube (16) comprising a tubular shaft (17), an inflatable sealing cuff (4)-including a collar (8a, 8b) at each end attached proximate to a patient end of said shaft (17) at spaced locations and a lumen (24) integrally formed along at least a portion of said shaft with a patient end located inside said cuff (4) for enabling fluid to be input from a machine end of the lumen (24) into said cuff (4) for inflating said cuff, **characterised in that** the cuff (4) has a smooth inner surface (14) and an outer surface (13) having attached at least one portion of a flash line resulting from the molding of the cuff (12), wherein, upon inverting the cuff, the smooth inner surface (14) of the cuff is presented externally of the tube (16) and said outer surface (13) of the cuff having the at least one portion of the flash line (12) is presented inwardly within the cuff (4).

7. Tracheal tube of claim 6, wherein said cuff is made from silicone by injection molding.

8. Tracheal tube of claim 6 or claim 7, wherein said tubular shaft is made from silicone.

9. Tracheal tube of any one of claims 6 to 8, further comprising a flange integrally formed as one piece with said shaft.

10. Tracheal tube of any one of claims 6 to 9, wherein the attachment of the outer surface of the cuff (4) having the flash line to the shaft (17) traps any material separating from the flash line within said cuff.

11. Tracheal tube of any one of claims 6 to 10, wherein said fluid is air or a liquid.

## Patentansprüche

1. Ein Verfahren zum Fertigen einer Trachealröhre, das die folgenden Schritte umfasst:
Gießen eines röhrenförmigen Schafts;
Gießen einer Manschette, die zwei Kragen beinhaltet, einen an jedem Ende der Manschette;
Invertieren der genannten Manschette, sodass ihre ursprüngliche innere Oberfläche die Oberfläche wird, die gegenüber der Umgebung exponiert ist, und ihre ursprüngliche äußere Oberfläche die innere Oberfläche der genannten Manschette bildet;
Anbringen der zwei Kragen der genannten Manschette nahe einem Patientenende des genannten Schafts an beabstandeten Stellen; und
einstückiges Bilden eines Lumens entlang mindestens eines Abschnitts des genannten Schafts, wobei sich dessen Patientenende innerhalb der genannten Manschette befindet, um der genannten Manschette zu ermöglichen, inflatiert zu werden, wenn ein Fluid in das Maschinenende des genannten Lumens eingegeben wird.

2. Verfahren nach Anspruch 1, wobei die genannte Manschette aus Silikon spritzgegossen wird.

3. Verfahren nach Anspruch 1 oder Anspruch 2, wobei der genannte Schaft aus Silikon spritzgegossen wird.

4. Verfahren gemäß einem der vorhergehenden Ansprüche, wobei die genannte Manschette an dem genannten Schaft durch Ankleben des Kragens an den genannten Schaft unter Verwendung eines flüssigen Silikon-RTV(Room Temperature Vulcanization, Raumtemperaturvernetzung)-Klebstoffs angebracht wird, wobei die angeklebte Manschette Material einschließt, das sich von einer Gratlinie trennen kann, die an der ursprünglichen äußeren Oberfläche der genannten Manschette gebildet wird.

5. Verfahren gemäß einem der vorhergehenden Ansprüche, das ferner den Schritt des einstückigen Gießens eines Flansches nahe einem Maschinenende des genannten Schafts umfasst, sodass der genannte Schaft und der genannte Flansch als einheitliches einzelnes Stück gebildet werden.

6. Eine Trachealröhre (16), die gemäß dem Verfahren nach einem der vorhergehenden Ansprüche gefertigt wird, wobei die Trachealröhre (16) Folgendes umfasst: einen röhrenförmigen Schaft (17), eine inflatierbare, abdichtende Manschette (4), einschließlich eines an jedem Ende nahe einem Patientenende des genannten Schafts (17) an beabstandeten Stellen angebrachten Kragens (8a, 8b), und ein Lumen (24), das entlang mindestens eines Abschnitts des genannten Schafts einstückig gebildet wird, wobei sich ein Patientenende innerhalb der genannten Manschette (4) befindet, um Fluid zu ermöglichen von einem Maschinenende des Lumens (24) in die genannte Manschette (4) eingegeben zu werden, um die genannte Manschette zu inflatieren, **dadurch gekennzeichnet, dass** die Manschette (4) eine glatte innere Oberfläche (14) und eine äußere Oberfläche (13), die mindestens einen an ihr angebrachten Abschnitt einer Gratlinie aufweist, die von dem Gießen der Manschette (12) resultiert, aufweist, wobei nach Invertieren der Manschette die glatte innere Oberfläche (14) der Manschette an der Außenseite der Röhre (16) präsentiert wird und die genannte äußere Oberfläche (13) der Manschette, die den mindestens einen Abschnitt der Gratlinie (12) aufweist, einwärts innerhalb der Manschette (4) präsentiert wird.

7. Trachealröhre nach Anspruch 6, wobei die genannte Manschette aus Silikon durch Spritzgießen hergestellt wird.

8. Trachealröhre nach Anspruch 6 oder Anspruch 7, wobei der genannte röhrenförmige Schaft aus Silikon hergestellt wird.

9. Trachealröhre nach einem der Ansprüche 6 bis 8, die ferner einen Flansch umfasst, der als ein Stück mit dem genannten Schaft einstückig gebildet wird.

10. Trachealröhre nach einem der Ansprüche 6 bis 9, wobei die Anbringung der äußeren Oberfläche der Manschette (4), die die Gratlinie aufweist, an den Schaft (17) Material einschließt, das sich von der Gratlinie innerhalb der genannten Manschette trennt.

11. Trachealröhre nach einem der Ansprüche 6 bis 10, wobei das genannte Fluid Luft oder eine Flüssigkeit ist.

## Revendications

1. Procédé de fabrication d'un tube trachéal, comprenant les étapes de :
moulage d'un arbre tubulaire ;
moulage d'un ballonnet incluant deux colliers, l'un à chaque extrémité du ballonnet ;
inversion dudit ballonnet de sorte que sa surface interne d'origine devienne la surface qui est exposée à l'environnement et sa surface externe d'origine forme la surface intérieure dudit ballonnet ;
fixation des deux colliers dudit ballonnet à proximité d'une extrémité patient dudit arbre au niveau d'emplacements espacés ; et
formation d'un seul tenant d'une lumière le long d'au moins une partie dudit arbre avec son extrémité patient située à l'intérieur dudit ballonnet pour permettre audit ballonnet d'être gonflé lorsqu'un fluide est entré dans l'extrémité machine de ladite lumière.

2. Procédé selon la revendication 1, dans lequel ledit ballonnet est moulé par injection à partir de silicone.

3. Procédé selon la revendication 1 ou la revendication 2, dans lequel ledit arbre est moulé par injection à partir de silicone.

4. Procédé selon l'une quelconque des revendications précédentes, dans lequel ledit ballonnet est fixé audit arbre par collage des colliers audit arbre au moyen d'un adhésif de RTV (vulcanisation à température ambiante) de silicone liquide, le ballonnet collé piégeant un matériau qui peut être séparé d'une ligne de bavure formée au niveau de la surface externe d'origine dudit ballonnet.

5. Procédé selon l'une quelconque des revendications précédentes, comprenant en outre l'étape de moulage d'un seul tenant d'une bride à proximité d'une extrémité machine dudit arbre de sorte que ledit arbre et ladite bride soient formés comme une seule pièce unitaire.

6. Tube trachéal (16) fabriqué selon le procédé selon l'une quelconque des revendications précédentes, le tube trachéal (16) comprenant un arbre tubulaire (17), un ballonnet d'étanchéité gonflable (4) incluant un collier (8a, 8b) au niveau de chaque extrémité fixé à proximité d'une extrémité patient dudit arbre (17) au niveau d'emplacements séparés et une lumière (24) formée d'un seul tenant le long d'au moins une partie dudit arbre avec une extrémité patient située à l'intérieur dudit ballonnet (4) pour permettre à un fluide d'être entré à partir d'une extrémité machine de la lumière (24) dans ledit ballonnet (4) pour gonfler ledit ballonnet, **caractérisé en ce que** le ballonnet (4) a une surface interne lisse (14) et une surface externe (13) à laquelle est fixée au moins une partie d'une ligne de bavure résultant du moulage du ballonnet (12), dans lequel, lors de l'inversion du ballonnet, la surface interne lisse (14) du ballonnet est présentée en externe du tube (16) et ladite surface externe (13) du ballonnet ayant l'au moins une partie de la ligne de bavure (12) est présentée vers l'intérieur à l'intérieur du ballonnet (4).

7. Tube trachéal selon la revendication 6, dans lequel ledit ballonnet est composé de silicone par moulage par injection.

8. Tube trachéal selon la revendication 6 ou la revendication 7, dans lequel ledit arbre tubulaire est composé de silicone.

9. Tube trachéal selon l'une quelconque des revendications 6 à 8, comprenant en outre une bride formée d'un seul tenant comme une seule pièce avec ledit arbre.

10. Tube trachéal selon l'une quelconque des revendications 6 à 9, dans lequel la fixation de la surface externe du ballonnet (4) ayant la ligne de bavure sur l'arbre (17) piège un quelconque matériau se séparant de la ligne de bavure à l'intérieur dudit ballonnet.

11. Tube trachéal selon l'une quelconque des revendications 6 à 10, dans lequel ledit fluide est de l'air ou un liquide.
